# EUROPEAN PATENT SPECIFICATION

(11) **EP 1 694 441 B1**
(45) Date of publication and mention of the grant of the patent: **21.09.2011**
(21) Application number: 04796883.9
(22) Date of filing: 29.10.2004
(51) Int. Cl.: B01J 35/10, B01J 23/28, B01J 23/70, B01J 23/16, B01J 23/06, C01B 3/58, C10K 3/04, H01M 8/06

(54) **Selective methanation of carbon monoxide in a hydrogen-rich fuel stream**
Selektive Methanizierung von Kohlenmonoxide in einem wasserstoffreichen Brennstoff
Méthanation sélective du monoxide de carbone présent dans un combustible constitué principalement d'hydrogène.

(30) Priority: 18.12.2003 US 740076
(43) Date of publication of application: 30.08.2006
(73) Proprietor: Süd-Chemie Inc., Louisville, KY 40210 (US)
(72) Inventor: HIROSHI, Takeda Rural Hayahoshi Apt. 202 737-2, Toyama,Fukushima 939-2706 (JP); WALSH, Troy, Louisville, KY 40218 (US); WAGNER, Jon, Louisville, KY 40241 (US)
(74) Representative: Stolmár Scheele & Partner
(86) International application number: PCT/US2004/036299
(87) International publication number: WO 2005/068077

(56) References cited:
- EP-A- 1 038 832
- EP-A- 1 201 300
- EP-A- 1 306 130
- WO-A-02/089978
- WO-A-2004/043596
- US-A- 4 490 481
- US-B1- 6 409 939
- PATENT ABSTRACTS OF JAPAN vol. 2002, no. 07, 3 July 2002 (2002-07-03) -& JP 2002 066321 A (IDEMITSU KOSAN CO LTD), 5 March 2002 (2002-03-05) -& JP 2002 066321 A (IDEMITSU KOSAN CO LTD) 5 March 2002 (2002-03-05)
- PATENT ABSTRACTS OF JAPAN vol. 2000, no. 16, 8 May 2001 (2001-05-08) & JP 2001 017861 A (TANAKA KIKINZOKU KOGYO KK), 23 January 2001 (2001-01-23)

## Description

### BACKGROUND OF THE INVENTION

### 1. Field of the Invention

The present invention is for a catalyst for the conversion of carbon monoxide. More specifically, this invention relates to catalyst comprising a support having a predetermined pore size and a metal capable of forming a metal carbonyl species. In one embodiment, the catalyst of the present invention comprises a mordenite, beta, or faujasite support and ruthenium metal.

### 2. Description of the Related Art

In a fuel cell, such as a Polymer Electrolyte Membrane Fuel Cell (PEMFC) stack, chemical energy of a fuel is converted into electrical energy. Typically, the fuel used is a hydrogen rich gas supplied to the fuel cell by a fuel processor. However, the gas from the fuel processor may further comprise unconverted hydrocarbon, water, carbon dioxide and carbon monoxide. The carbon monoxide, in particular, is detrimental to the PEMFC stack because the carbon monoxide can poison the noble metal electrodes utilized by the fuel cells, thereby reducing the electrical output.

Preferably, the CO concentration for a fuel cell feed should be at a level below about 100 ppm, and more preferably to a level of less than about 50 ppm. However, as received from the fuel processor, the CO concentrations may be in excess of about 1 wt%, thus requiring further reduction of CO concentration. Some typical methods for reducing the CO concentration include selective catalytic oxidation of CO, pressure swing adsorption, hydrogen separation by membrane, and selective methanation of CO.

Selective catalytic oxidation of CO (Eq. 1) is a well-known process for reducing the CO concentration for fuel cells. But, oxidation of hydrogen (Eq. 2) is a competitive reaction.

½ O₂ + CO → CO₂ Eq. 1

½ O₂ + H₂ → H₂O Eq. 2

Thus, in order to maximize the concentration of hydrogen gas and minimize the concentration of carbon monoxide, it is necessary to have reaction conditions wherein Eq. 1 is favored over Eq. 2. One option for achieving this is to have a highly specific catalyst for the oxidation of carbon monoxide and to limit the oxygen concentration so that the oxygen is consumed primarily for the production of carbon dioxide. Theoretically, this is achievable, but in practice there are wide swings in the CO concentrations produced by the fuel processor and it can be difficult to adjust the oxygen input to track the CO concentration. Because the CO is more detrimental to the fuel cell than water, it is typical for excess oxygen to be fed into the reactor thereby essentially ensuring that the CO will be converted to CO₂. The disadvantage is that significant quantities of H₂ are converted to water by operating in this manner.

A method for the catalytic selective oxidation of carbon monoxide in a hydrogen-rich fuel stream, wherein the catalyst is similar to the catalyst used in the carbon monoxide methanation of the present invention is known from US 6 409 939 B1.

Pressure swing adsorption is an industrially proven technology, but it requires relatively high pressure operation. Thus, while this process may be effective for use in larger fuel cells, it is not practical at this time for smaller fuel cells.

Hydrogen separation by membrane is effective for separating hydrogen from carbon monoxide. But the process requires a substantial pressure drop to effect the separation, and the cost and durability of the membranes still must be proven.

Selective methanation (Eq. 3) is a process whereby carbon monoxide is reacted with hydrogen in the presence of a catalyst to produce methane and water and methanation of carbon dioxide is minimized. Commonly used in ammonia plants, total carbon oxide methanation is known to reduce carbon monoxide and carbon dioxide concentrations to levels as low as about 5 ppmv to 10 ppmv, and the industrial catalysts are not selective. However, in most fuel cell applications, the selective methanation reaction is accompanied by a reverse water-gas-shift reaction (Eq. 4), which also is generally facilitated by a catalyst. Thus, while the CO concentration is being reduced through methanation, additional carbon monoxide is formed from the carbon dioxide present to maintain the equilibrium of the water-gas-shift reaction.

CO + 3H₂ → CH₄ + H₂O Eq. 3

CO₂ + H₂ ↔ CO + H₂O Eq. 4

Under the proper reaction conditions and with a non-selective methanation catalyst, the CO₂ may be methanated as shown in Eq. 5.

CO₂ + 4H₂ →CH₄ + 2H₂O Eq. 5

But, this is generally an undesirable reaction because it further consumes H₂ and the CO₂ methanation is normally accompanied by a temperature rise in the reactor that can lead to "run-away" conditions. Considering that the carbon dioxide concentration is greater than 10 times that of carbon monoxide, achieving selectivity is not thermodynamically favorable. Thus, it would be advantageous to have a catalyst that is highly selective for CO methanation, essentially suppresses CO₂ methanation and does not facilitate the conversion of CO₂ to CO through the water-gas-shift reaction.

In the prior art methanation processes, precious metals supported on non-zeolitic materials, such as Al₂O₃, SiO₂, and TiO₂, have been used as catalysts in the selective methanation of CO (see, for example, U.S. Pat. No. 3,615,164 and U.S. Pat. Pub. No. 2003/0086866). For example, in Patent Number WO 01/64337, ruthenium (Ru) on a carrier base support of Al₂O₃, TiO₂, SiO₂, ZrO₂, or Al₂O₃-TiO₂ with egg-shell structure is taught to reduce the CO to concentrations of about 800 ppm with 70-80% selectivity under an atmosphere of CO at 0.6%, CO₂ at 15%, H₂ at 64.4%, H₂O at 20% and GHSV = 10,000 H⁻¹. However, for an efficient PEMFC power system, the CO concentration should be less than about 100 ppm, and preferably equal to or less than about 50 ppm. Since the CO concentration from the selective methanation processes using the prior art catalysts are significantly higher than the desired maximum concentration for a PEMFC stack, these catalysts cannot be practically used in PEMFC power systems.

Thus, it would be advantageous to have a catalyst that is highly selective for CO methanation, essentially suppresses CO₂ methanation and does not facilitate the conversion of CO₂ to CO through the water-gas-shift reaction.

### SUMMARY OF THE INVENTION

The catalyst useful for the carbon monoxide methanation reactions according to the invention comprises a metal capable of forming a metal-carbonyl species on a support having a predetermined pore size. The catalyst comprises a metal selected from the group consisting of ruthenium, rhodium and nickel, which form a metal-carbonyl species on a support selected from the group consisting of beta-zeolite, mordenite and faujasite, and having a regular lattice structure and a predetermined pore diameter of sufficient dimensions to accommodate the carbonylated metal species. In an embodiment, the metal is ruthenium and the support is selected from mordenite, beta-zeolite or faujasite and has a pore diameter of greater than about 6.3Å, and a pore volume in the range of from about 0.3cm³/g to about 1.0cm³/g. An inert binder, such as alumina, γ-Al₂O₃, SiO₂, ZrO₂, TiO₂ or pseudo-boehmite, may optionally be added to the catalyst. The catalyst efficiently facilitates the selective hydrogenation of carbon monoxide using H₂ that is present in the reformate and reduces the concentration of the CO to levels equal to or less than about 50 ppm.

The present invention further includes a process for CO "polishing", whereby the concentration of CO in a mixture of gases containing hydrogen, hydrocarbons, carbon dioxide, carbon monoxide and water is removed or substantially reduced. Particularly, this invention is directed to a method of selective methanation whereby carbon monoxide is reduced to a concentration level such that the residual hydrogen is suitable for use as a fuel in a fuel cell and the overall efficiency of the PEMFC power system is improved.

### DETAILED DESCRIPTION OF THE PREFERRED EMBODIMENT

The catalyst has demonstrated benefits in facilitating the carbon oxide methanation reactions in small fuel cells. In general terms, the catalyst comprises a metal capable of forming a metal-carbonyl species on a support having a predetermined pore size of sufficient dimensions to allow the pore to accommodate a fully carbonylated metal complex. As is known in the art, some typical supports for catalysts are crystalline alumino-silicate materials. Among the metals known in the art to form stable metal-carbonyl complexes are ruthenium, rhodium and nickel. Optionally, an inert binder, such as alumina, γ-Al₂O₃, SiO₂, ZrO₂, TiO₂ or pseudo-boehmite, may optionally be added to the catalyst.

The present invention will be described herein through, without limitation, exemplary embodiments, figures and examples. Any embodiments, figures, examples and related data presented herein are merely to exemplify the principles of the invention, and are not intended to limit the scope of the invention.

The support of the catalyst of the present invention comprises a crystalline alumino-silicate having a predetermined pore size. The crystalline alumino-silicate is a molecular sieve selected from beta-zeolite, mordenite and faujasite. Because it is believed that the methanation reaction occurs within the support pore, the pore must be of sufficient dimensions to accommodate a fully carbonylated metal complex, and thus, the pore size requirement will vary depending on the metal species selected for the catalyst. However, it has generally been observed that if the pore size is smaller than or is significantly larger than the dimensions of the fully carbonylated metal species, the resulting catalyst does not show the desired selectivity for carbon monoxide methanation.

The metal of the catalyst must be capable of forming a metal-carbonyl species. As is known in the art, metals may form metal-carbonyl complexes wherein each ligand is a carbonyl unit, or metals may form metal-carbonyl complexes wherein at least one ligand is not a carbonyl. For the purpose of the development, it is not necessary that the metal be capable of forming a fully-carbonylated complexes, e.g. wherein each ligand is a carbonyl group. Rather, a "fully-carbonylated" complex - for the purpose of calculating the volume needed within the support pore- is defined herein as the metal complex with the maximum number of carbon monoxide ligands that the metal prefers to accommodate in its lowest energy state. The metal is preferably selected from the group consisting of ruthenium, rhodium and nickel. As delivered to the catalyst, the metal may be a base metal or it may be a metal oxide complex.

The metal may be added to the support by any means known in the art for intercalating the metal into the support pores, such as, without limitation, impregnation, incipient wetness method, immersion and spraying. The embodiments presented herein add the metal through impregnation for exemplary purposes only. Although not a requirement to practice the invention, it is recommended that the metal source be free of typically recognized poisons, such as sulfur, chlorine, sodium, bromine, iodine or combinations thereof. Acceptable catalyst can be prepared using metal sources that include such poisons, but care must be taken to wash the poisons from the catalyst during production of the catalyst.

In an embodiment of the present invention, the support is a crystalline alumino-silicate selected from mordenite, beta-zeolite or faujasite. The support has a pore diameter of greater than about 6.3Å, and a pore volume in the range of from about 0.3cm³/g to about 1.0cm³/g, and preferably in the range of 0.5cm³/g to about 0.8cm³/g. Ruthenium is impregnated on the support so as to deliver a concentration of from about 0.5 wt% Ru to about 4.5 wt% Ru, based on the total weight of the catalyst including the ruthenium. Some recommended sources of ruthenium include, without limitation, Ru(NO)(NO₃)ₓ(OH)_{y}, Ru(NO₂)₂(NO₃)₂, Ru(NO₃)₃, RuCl₃, Ru(CH₃COO₃), (NH₄)₂RuCl₆, [Ru(NH₃)₆]Cl₃, Ru(NO)Cl₃, and Ru₃(CO)₁₂. Optionally, the catalyst further comprises the binder γ-Al₂O₃ at a loading of about 20 wt%, including the weight of the binder. The catalyst may be used in an exemplary process for removing or substantially reducing the quantity of carbon monoxide in a mixture of gases containing hydrogen, carbon dioxide, carbon monoxide, and water. The process involves passing a mixture of gases over the catalyst in a reaction zone having a temperature below the temperature at which the shift reaction occurs and above the temperature at which the selective methanation of carbon monoxide occurs.

It is understood that variations may be made which would fall within the scope of this development. For example, although the catalysts of the present invention are intended for use as selective methanation catalysts for the conversion of carbon monoxide for fuel cell applications, it is anticipated that these catalysts could be used in other applications requiring highly selective carbon oxide methanation catalysts.

## Claims

1. Use of a catalyst comprising a metal selected from the group consisting of ruthenium, rhodium and nickel, on a support selected from the group consisting of beta-zeolite, mordenite and faujasite, for carbon monoxide methanation reactions.

2. The use according to Claim 1 wherein the metal is ruthenium.

3. The use according to Claim 1 or 2 further comprising an inert binder.

4. The use according to Claim 3 wherein the binder is selected from the group consisting of alumina, y-Al₂O₃, SiO₂, ZrO₂, TiO₂ or pseudo-boehmite.

5. The use according to any of Claims 1-4 wherein the metal is added to the support through impregnation, incipient wetness method, immersion, or spraying.

6. The use according to any of Claims 2-5 wherein the ruthenium is added to the support through impregnation.

7. The use according to Claim 2 wherein the support has a pore volume in the range of from 0.3 cm³/g to 1.0 cm³/g.

8. The use according to Claim 7 wherein the metal is ruthenium impregnated on the support so as to deliver a concentration of from 0.5 wt% Ru to 4.5 wt% Ru, based on the total weight of the catalyst including the ruthenium.

9. The use according to Claim 7 wherein the support has a pore diameter of greater than 6.3 angstroms.

10. The use according to Claim 8 wherein the catalyst further comprises the binder y-Al₂O₃ at a loading of 20 wt%, including the weight of the binder.

## Patentansprüche

1. Verwendung eines Katalysators, umfassend ein Metall, ausgewählt aus der Gruppe bestehend aus Ruthenium, Rhodium und Nickel, auf einem Träger, ausgewählt aus der Gruppe bestehend aus Beta-Zeolith, Mordenit und Faujasit für Kohlenmonoxidmethanisierungsreaktionen.

2. Verwendung nach Anspruch 1; wobei das Metall Ruthenium ist.

3. Verwendung nach Anspruch 1 oder 2, weiterhin umfassend ein inertes Bindemittel.

4. Verwendung nach Anspruch 3, wobei das Bindemittel ausgewählt ist aus der Gruppe, bestehend aus Aluminiumoxid, γ-Al₂O₃, SiO₂, ZrO₂, TiO₂ oder Pseudo-Boehmit.

5. Verwendung nach einem der Ansprüche 1-4, wobei das Metall dem Träger durch Imprägnierung, Anfeuchtungsverfahren, Eintauchen oder Sprühen zugegeben wird.

6. Verwendung nach einem der Ansprüche 2-5, wobei das Ruthenium dem Träger durch Imprägnierung zugegeben wird.

7. Verwendung nach Anspruch 2, wobei der Träger ein Porenvolumen im Bereich von 0,3 cm³/g bis 1,0 cm³/g aufweist.

8. Verwendung nach Anspruch 7, wobei das Metall Ruthenium ist, mit dem der Träger so imprägniert wird, dass eine Konzentration von 0,5 Gewichts-% Ru bis 4,5 Gewichts-% Ru, basierend auf dem Gesamtgewicht des Katalysators einschließlich Ruthenium, abgegeben wird.

9. Verwendung nach Anspruch 7, wobei der Träger einen Porendurchmesser von mehr als 6,3 Ångström aufweist.

10. Verwendung nach Anspruch 8, wobei der Katalysator weiterhin das Bindemittel γ-Al₂O₃ mit einer Beladung von 20 Gewichts-% einschließlich des Gewichts des Bindemittels umfasst.

## Revendications

1. Utilisation d'un catalyseur comprenant un métal sélectionné du groupe consistant du ruthénium, du rhodium et du nickel sur un support sélectionné du groupe consistant de la zéolithe bêta, de la mordénite et de la faujasite pour des réactions de méthanation du monoxyde de carbone.

2. Utilisation selon la revendication 1, où le métal est le ruthénium.

3. Utilisation selon la revendication 1 ou 2, qui comprend également un liant inerte.

4. Utilisation selon la revendication 3, où le liant est sélectionné du groupe suivant : oxyde d'aluminium, y-Al₂O₃, SiO₂, ZrO₂, TiO₂ ou pseudo-boehmite.

5. Utilisation selon l'une quelconque des revendications 1-4, où le métal est déposé sur le support par imprégnation selon la méthode à humidité naissante, par immersion ou par pulvérisation.

6. Utilisation selon l'une quelconque des revendications 2-5, où le ruthénium est déposé sur le support par imprégnation.

7. Utilisation selon la revendication 2, où le volume poreux du support est compris entre 0,3 cm³/g et 1,0 cm³/g.

8. Utilisation selon la revendication 7, où le métal est le ruthénium imprégné sur le support de façon à obtenir une concentration comprise entre 0,5 % en poids de Ru et 4,5 % en poids de Ru sur la base du poids total du catalyseur, y compris du ruthénium.

9. Utilisation selon la revendication 7, où le diamètre des pores du support est supérieur à 6,3 angströms.

10. Utilisation selon la revendication 8, où le catalyseur comprend également le liant qu'est y-Al₂O₃ à un taux de 20 % en poids, y compris le poids du liant.
